# EUROPÄISCHE PATENTSCHRIFT

(11) **EP 3 306 304 B1**
(45) Veröffentlichungstag und Bekanntmachung des Hinweises auf die Patenterteilung: **02.06.2021**
(21) Anmeldenummer: 17191538.2
(22) Anmeldetag: 18.09.2017
(51) Int. Cl.: G01N 21/51, G01J 3/02, G01J 3/44, G01N 33/14, G01N 21/65, G02B 21/34, G02B 26/02

(54) **VERFAHREN UND VORRICHTUNG ZUR ANALYSE VON ALKOHOLISCHEN GETRÄNKEN**
METHOD AND APPARATUS FOR ANALYZING ALCOHOLIC BEVERAGES
MÉTHODE ET APPAREIL D'ANALYSE DES BOISSONS ALCOOLISÉES

(30) Priorität: 26.09.2016 DE 102016118080
(43) Veröffentlichungstag der Anmeldung: 11.04.2018
(73) Patentinhaber: WhiskyAuction.Com GbR, 22337 Hamburg (DE)
(72) Erfinder: Rosenfeld, Klaus, 22337 Hamburg (DE)
(74) Vertreter: Limbeck, Achim

(56) Entgegenhaltungen:
- EP-A2- 1 560 058
- JP-A- 2006 266 948
- US-A- 4 325 083
- US-A- 5 377 004
- US-A- 5 859 703
- US-A1- 2009 146 061
- US-A1- 2011 292 376
- US-A1- 2014 319 356
- US-A1- 2016 123 876
- NORDON A ET AL: "Comparison of non-invasive NIR and Raman spectrometries for determination of alcohol content of spirits", ANALYTICA CHIMICA ACTA, ELSEVIER, AMSTERDAM, NL, Bd. 548, Nr. 1-2, 29. August 2005 (2005-08-29), Seiten 148-158, XP027730890, ISSN: 0003-2670 [gefunden am 2005-08-29]
- MARTIN CORALIE ET AL: "Raman spectroscopy of white wines", FOOD CHEMISTRY, ELSEVIER LTD, NL, Bd. 181, 20. Februar 2015 (2015-02-20), Seiten 235-240, XP029208825, ISSN: 0308-8146, DOI: 10.1016/J.FOODCHEM.2015.02.076
- ELIASSON ET AL: "Non-invasive detection of cocaine dissolved in beverages using displaced Raman spectroscopy", ANALYTICA CHIMICA ACTA, ELSEVIER, AMSTERDAM, NL, Bd. 607, Nr. 1, 19. November 2007 (2007-11-19), Seiten 50-53, XP022394460, ISSN: 0003-2670, DOI: 10.1016/J.ACA.2007.11.023

## Beschreibung

### Technisches Gebiet

Die vorliegende Erfindung betrifft ein Verfahren gemäß dem Oberbegriff des Patentanspruchs 1. Ferner betrifft die vorliegende Erfindung eine Vorrichtung zur Durchführung dieses Verfahrens gemäß Anspruch 3.

### Stand der Technik

Gemäß dem Stand der Technik sind Vorrichtungen zur Qualitätskontrolle alkoholischer Getränke sowie zur quantitativen Bestimmung des Alkoholgehaltes in alkoholischen Getränken bereits bekannt, welche mit unterschiedlichen spektroskopischen Techniken arbeiten, wie bspw. die H-NMR (H-Kernresonanzspektroskopie) oder die Nah-Infrarot-Spektroskopie.

Bei der H-NMR-Spektroskopie wird die Radioabsorption des Wasserstoffisotops 1H gemessen, wobei die Intensität dieser Absorption für alle 1H-Atome - auch in unterschiedlichen Molekülen - gleich ist. Indem die Absorptionssignale von 1H in Wasser und Alkohol miteinander ins Verhältnis gesetzt werden, wird auf diese Weise die Alkoholkonzentration einer wässrigen Probe bestimmt. Hierzu kann bspw. ein 1H-NMR-Spektrometer mit einer Protonenresonanzfrequenz von 600 MHz eingesetzt werden.

Im Rahmen der Nah-Infrarot-Spektroskopie nach Praveen C. Ashok, Bavishna B. Praveen, und K. Dholakia, "Near infrared spectroscopic analysis of single malt Scotch whisky on an optofluidic chip", Optics Express 19(23), 2011, erfolgt die Qualitätsüberwachung von alkoholischen Getränken im nahen Infrarotbereich durch eine laserbasierte Raman-Spektroskopie mit einem optofluidischen Chip, mittels dem eine relativ fehlerfreie und schnelle Analyse von alkoholischen Getränken mit drastisch reduzierten Probenvolumina ermöglicht werden soll.

Nachteilig bei den vorgestellten Verfahren und Vorrichtungen ist, dass die Analyse eines alkoholischen Getränks innerhalb eines geschlossenen flaschenartigen Behältnisses (bspw. einer Flasche Whisky) nicht möglich ist, da zur Analyse des Alkohols eine flüssige Probe vorliegen muss, welche dem Behältnis zunächst zu entnehmen ist. Hierzu ist es allerdings erforderlich, das Behältnis zu öffnen bzw. dieses - im Zweifel irreversibel - zu zerstören.

Die Druckschriften US 2004/0004 194 A1, der US 6 879 394 B2 sowie aus der WO 2017/020936 A1 beschäftigen sich mit einem Raman-Aufbau zur Untersuchung beliebiger Substanzen mittels einer Scanning-Raman-Mikroskopie, bei dem ein Makro-Objektiv zum Einsatz kommt. Allerdings sind die in den genannten Druckschriften nicht speziell zur Untersuchung von alkoholischen Getränken innerhalb einer Flasche ausgebildet. Es fehlen außerdem ein motorischer Filter mit unterschiedlicher Dichte und andere konstruktive Merkmale, die sich für diesen Anwendungszweck einsatzfähig machen würden.

Weiterhin ist in der EP 0 606 639 B1 ein Verfahren dargestellt, mit dem Kunststoffflaschen durch Raman-spektrometrische Methoden untersucht werden können. Allerdings betrifft diese Untersuchungsmethode nur die Kunststoffflaschen selbst, jedoch nicht seinen Inhalt wie bspw. alkoholische Getränke. Es fehlen weiterhin konstruktive Merkmale wie ein Mikroskop oder ein Makro-Objektiv.

In der US 2011/292376 A1 ist eine Vorrichtung und ein Verfahren zum Detektieren von Raman- und Photolumineszenzspektren einer Substanz und zum Identifizie-ren der Substanz durch Raman und/oder Photolumineszenzspektraleigenschaften der Substanz offenbart, ein ersetzbares Laserquellenaggregat mit einer Laserquelle, ein Kollimationssystem und eine Fassung zum Aufnehmen des ersetzbaren Laserquellenaggregats umfasst, während der Betrieb der Vorrichtung ohne weitere Einstellung einer Positionierung des Kollimatorsystems oder der Laserquelle sichergestellt wird. Es sind weiterhin ein Filtersystem sowie ein Lichtdispersionssystem vorge-sehen, das für eine spektrale Auflösung optimiert ist, und einen Spektralbereich umfasst, der ausreicht, um gleichzeitig Raman- und Photolumineszenzspektren der Substanz zu erhalten. Ein Detektor sowie mindestens eine Steuerung zum Verarbeiten elektri-scher Signale sind ebenfalls vorgesehen. Das offenbarte und beanspruchte Verfahren sieht das gleichzeitige Erhalten von Raman- und Photolumineszenzspektren einer Substanz zum Trennen der Spektren in Komponenten auf der Grundlage von Raman- und Photolumineszenzgehalten zum Analysieren des Raman- und Photolumineszenzgehalts und zum Identifizieren der Substanz unter Verwendung eines Satzes spektraler Verarbeitung vor.

Die US 4 325 083 A beschreibt ein System, bei dem multispektrale Filterung und variable optische Dämpfung auf derselben Unterlage ausgeführt werden. Im Falle einer Stützscheibe hat sie zwei winkelförmige abgestufte Dämpfungsglieder, die symmetrisch bezüglich eines referenzierten Durchmessers hergestellt sind. Jeder Abschwächer bildet auch optische Filter in einem gegebenen Spektralband, um in zwei getrennten Bändern arbeiten zu können. Die Scheibe ist in der Nähe eines Relaisobjektivs angeordnet, das zwischen dem Empfangsobjektiv und dem optoelektrischen Wandler angeordnet ist. Das detektierte Signal liefert eine Schleife, die die Winkelposition der Platte steuert. Die Scheibe wird so gesteuert, dass das Schalten des Bandes beim Passieren der Zonen maximaler Dichte der Dämpfer stattfindet.

Die US 2016/123876 A beschreibt ein System mit einer Computervorrichtung, die einen Speicher umfasst, der zum Speichern von Anweisungen konfiguriert ist. Die Rechenvorrichtung umfasst auch einen Prozessor zum Ausführen der Anweisungen zum Ausführen von Vorgängen, einschließlich Initiieren der Übertragung von einfallendem Licht von einer oder mehreren Lichtquellen zu einer versiegelten Flasche, die Flüssigkeit enthält. Die Vorgänge umfassen auch das Empfangen von Streulicht von der in der verschlossenen Flasche enthaltenen Flüssigkeit. Die Operationen umfassen auch das Verarbeiten eines oder mehrerer Signale, die für das gestreute Licht repräsentativ sind, um Wechselwirkungen des einfallenden Lichts mit einem bestimmten Molekül zu detektieren.

In der Veröffentlichung NORDON ET AL in Analytica Chimica Acta, Bd. 548, Nr. 1-2 (2005-08-29), XP027730890, ISSN: 0003-2670 wurde die Eignung von nichtinvasiven NIR- und Raman-Spektrometrien zur Bestimmung des prozentualen Ethanolgehaltes untersucht. Hierbei wurden Proben von Whisky, Wodka und zuckerhaltigen alkoholischen Getränken wurden in 200 ml (flach) und 700 ml (rund) Glasflaschen analysiert.

In der Veröffentlichung "Non-invasive detection of cocaine dissolved in beverages using displaced Raman spectroscopy" von C. Eliasson, N.A. Macleod und P. Matousek wird das Potenzial der Raman-Spektroskopie zum Nachweis von Kokain dargestellt, wobei diese in transparenten Glasflaschen mit alkoholischen Getränken versteckt sind. Die Technik verspricht einen schnellen, nicht-invasiven Nachweis verdeckter illegaler Substanzen in Getränken mit tragbaren Raman-Instrumenten.

Aus der EP 1 560 058 A2 ist ein optisches System bekannt, das eine kontinuierliche Betrachtung eines vergrößerten Bildes einer Probe und eine gleichzeitige Infrarot-Spektralanalyse ermöglicht. Dieses Sytem umfasst ein internes Reflexionselement mit einer Oberfläche, die geeignet ist, eine Probe zu kontaktieren, und die einen ersten optischen Pfad bereitstellt. Darüber hinaus ist eine Linse vorgesehen, die derart ausgebildet und positioniert ist, dass sie ein vergrößertes Bild der Probe auf einer oder mehreren Reihen von Photodetektoren erstellen kann.

Die US 5 4- 377 004 A betrifft eine holographische Sonde, die es ermöglicht, die Messung von Strahlungseffekten wie Raman-Streuung oder Fluoreszenz einer entfernt angeordneten Probe vorzunehmen. Verbessert nach dem Stand der Technik, lehrt diese Sonde einen im Wesentlichen inline Weg zwischen der Probe und einer Ausgangsoptik, wobei ein schmalbandiges reflektierendes Element, vorzugsweise holographisch aufgezeichnet, verwendet wird, um Anregungsenergie von einem Beleuchtungspfad in den Sammelpfad zu falten und jede Rayleigh-Streuung, die von der Probe empfangen wird, abzulehnen.

Die Schriften JP 2006 266948 A, US 2009/0146061 A1, US 5 859 703 A und US 2014/319356 A1 beschreiben Halterungen und/oder Positionierungen von flaschenartigen Behältnissen für optische Untersuchungen von darin enthaltenen Flüssigkeiten.

### Darstellung der Erfindung

Der vorliegenden Erfindung liegt die Aufgabe zu Grunde, ein Verfahren zu schaffen, welche die vorgenannten Probleme ausräumt und welche geeignet ist, ein alkoholisches Getränk innerhalb eines geschlossenen Behältnisses zu analysieren.

Erfindungsgemäß wird die voranstehende Aufgabe gemäß Anspruch gelöst. Vorteilhafte Ausgestaltungen und Weiterbildungen des erfindungsgemäßen Verfahrens sind in den abhängigen Unteransprüchen angegeben.

Eine stufenlose Höhenverstellung, möglicherweise motorisiert, bestimmt, wie tief der Mess- und Brennpunkt innerhalb des Glases oder der Flüssigkeit liegt. Per Umschaltung kann das Mikroskop auch, wie ein normales Mikroskop, mit sichtbarem Licht genutzt werden, zur optischen Untersuchung der Probe z.B. der Glasoberfläche. Somit kann man den Punkt der Höhenverstellung finden und die Lage des Glases bestimmen und für die folgende Lasereinstellung nutzen.

Durch die Verwendung eines Macro-Objektivs sowie der Anordnung des flaschenartigen Behältnisses direkt unterhalb des Objektives ist es möglich, eine Messung im Objektiv-Brennpunkt vorzunehmen, welche innerhalb des flaschenartigen Behältnisses liegt, wobei eine Interpretation entweder der Einzelspektren oder der Graphen der Hauptkomponentenanalyse erfolgen kann. Dabei kann sowohl eine Stelle am Flaschenbauch sowie auch eine Stelle am Flaschenhals genutzt werden. Die Messung durch eine Luftblase ist zu vermeiden.

Dadurch wird eine Analyse des in dem flaschenartigen Behältnis vorhandenen Inhalts ermöglicht, ohne dieses zuvor aus der Flasche entnehmen zu müssen. Das flaschenartige Behältnis bleibt somit während der Analyse geschlossen und mithin unversehrt. Um eine vergleichende Analyse vorzunehmen, sind vorzugsweise Vergleichsflaschen vorhanden, welche im Wechsel mittels des erfindungsgemäßen Verfahrens analysiert werden.

Dies wird ermöglicht durch den Einsatz eines Macro-Objektivs mit einer langen Brennweite, mindestens d=10mm, um auch weiter im Flascheninneren messen zu können.

In einer weiteren Ausführungsform des erfindungsgemäßen Verfahrens kann der Brennpunkt alternativ auch in das Glas des flaschenartiges Behältnisses gebracht werden, wobei ein Spektrum des Flaschenglases gewonnen wird, das zur Differenzbildung vom Gesamtspektrum abgezogen werden kann.

Erfindungsgemäß ist ein Laser vorzusehen, eine Wellenlänge von 785nm umfasst. Zudem kann bei jeder Messung eine Auswahl der passenden Laser-Intensität sowohl für die Glasmessung als auch die Flüssigkeitsmessung erfolgen.

Zur Durchführung des Verfahrens wird wie folgt vorgegangen: Zunächst werden zwei verschlossene Behältnisse mit Alkoholinhalt nacheinander mit dem Raman-Spektroskop vermessen. Die gemessenen Spektren werden miteinander verglichen, mit Hilfe einer Hauptkomponentenanalyse. Beim Ergebnis sehr vieler Übereinstimmungen der beiden Spektren ist der Inhalt der Behältnisse gleich, welches die Echtheit des Inhalts beweist. Sofern sehr wenige Übereinstimmungen der beiden Spektren vorliegen, ist der Inhalt als unterschiedlich anzusehen, so dass eine der beiden Inhalte vermutlich gefälscht ist.

### Kurzbeschreibung der Zeichnung

Weitere Ziele, Merkmale, Vorteile und Anwendungsmöglichkeiten des erfindungsgemäßen Verfahrens ergeben sich aus der nachfolgenden Beschreibung eines Ausführungsbeispiels anhand der Zeichnung. Dabei bilden alle beschriebenen und/oder bildlich dargestellten Merkmale für sich oder in beliebiger Kombination den Gegenstand der Erfindung, unabhängig von der Zusammenfassung in einzelnen Ansprüchen oder deren Rückbeziehung.

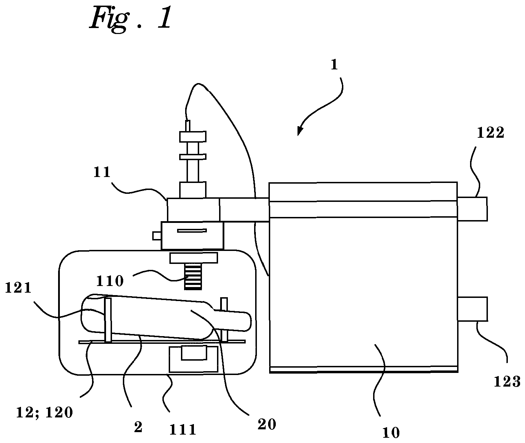

In der Zeichnung zeigt
Fig.1 die erfindungsgemäße Vorrichtung in einer schematischen Frontansicht.

### Ausführung der Erfindung

Wie aus Fig.1 ersichtlich, umfasst die erfindungsgemäße Vorrichtung 1 zur Durchführung des Verfahrens ein Raman-Spektrometer 10 zur Detektion eines Raman-Spektrums eines zu untersuchenden Mediums mit mindestens einer optischen Einrichtung zur Erzeugung eines Laserstrahls sowie ferner ein Mikroskop 11 mit einer Halterung 12 für das zu untersuchende Medium.

Die Halterung 12 ist zur Aufnahme eines flaschenartigen Behältnisses 2 ausgebildet und umfasst in dieser vorteilhaften Ausführungsform eine plattenförmige Aufnahme 120 zur Ablage des Behältnisses 2 sowie besonders vorteilhaft das Behältnis 2 umfassende, flexible Halter 121, welche zur Fixierung des Behältnisses ausgelegt sind.

Ganz besonders vorteilhaft kommt ein sog. Stigmatic Single-pass Spektrometer mit einem hohen Durchsatz von RAMAN Streulicht von Probe zum Detektor zum Einsatz. Der Durchmesser des Laserpunktes auf der Probenoberfläche ist vorzugsweise kontinuierlich variabel von 1 µm bis 300 µm, abhängig von Objektiv und Anregungswellenlänge, mit vollständig optimiertem Strahlenpfad.

Überdies ist es vorteilhaft, motorisierte Linsen für Licht im sichtbaren Spektrum für optimale spektrale Auflösung bei Verwendung unterschiedlicher Anregungswellenlängen sowie einen kontinuierlich einstellbaren "Easy Confocal" Modus mit motorisiertem Schlitz und automatisierter Optimierung des Signals vorzusehen.

Die erfindungsgemäße Vorrichtung 1 umfasst ferner einen "extended scanning" Modus zur kontinuierlichen Aufnahme von Spektren mit höchstmöglicher Auflösung über einen weiten Spektralbereich. Es werden keine Einzelspektren zusammengesetzt, sondern die Aufnahme erfolgt kontinuierlich (kein "Stitching"). Über ein CCD-Binning kann die spektrale Auflösung vorzugsweise kontinuierlich variiert werden.

Gemäß der Erfindung ist ein motorisierter Filter unterschiedlicher optischer Dichte zur Einstellung der Laserintensität auf der Probe vorgesehen, zum Beispiel (Neutral Density Filter) von 0.00005 bis 100%, wobei die spektrale Auflösung vorzugsweise zwischen < 1 und 2 cm-1 liegt, abhängig von gewählter Laser-Wellenlänge und Gitter.

Das Mikroskop 11 ist von einer Schalung 111 umgeben.

Es umfasst erfindungsgemäß ein Macro-Objektiv 110 mit einer langen Brennweite von mindestens 10mm. Bestenfalls ist dieses für Messungen mit einer Tiefenauflösung von 1 µm ausgelegt. Die Beleuchtung erfolgt hier vorzugsweise durch reflektiertes Licht.

Ganz besonders vorteilhaft weist das Mikroskop einen Monocularkopf und eine Farb-Videokamera auf.

Zur Aufnahme und Auswertung der mittels der Vorrichtung ermittelten Daten kommt eine Software zum Einsatz, welche bis zu 50 Mio. Einzelspektren in einem Mapping File verarbeiten kann. Die Software beinhaltet überdies: Aufnahme und Darstellung von Weißlichtbildern (image capture); Gleichzeitige Visualisierung von Probe und Lasermesspunkt; Automatische Umschaltung zwischen Visualisierung und Ramanmessmodus; Digitale Speicherung der Bilder; flexibel und einfach zu bedienen; die Daten sind exportierbar. Für die visuelle Darstellung der Ergebnisse und mit chemometrischen Methoden für die Auswertung der gemessenen Daten kommt vorzugsweise ein Chemometrics Softwarepaket zum Einsatz.

Das erfindungsgemäße Verfahren beschränkt sich in seiner Ausführung nicht auf die vorstehend angegebenen bevorzugten Ausführungsformen. Vielmehr sind, limitiert nur durch die angehängten Ansprüche, eine Vielzahl von Ausgestaltungsvariationen denkbar, welche von der dargestellten Lösung auch bei grundsätzlich anders gearteter Ausführung Gebrauch machen.

### Liste der Bezugsziffern

- 1: Vorrichtung
- 2: flaschenartiges Behältnis
- 10: Spektrometer
- 11: Mikroskop
- 12: Halterung
- 20: Probe innerhalb des flaschenartigen Behältnisses
- 110: Objektiv
- 111: Schalung
- 120: plattenförmige Aufnahme
- 121: Halter
- 122: Kamera
- 123: Laser

## Patentansprüche

1. Verfahren zur Analyse von alkoholischen Getränken zu deren Echtheitsprüfung mittels eines Raman-Spektrometers (10) zur Detektion eines Raman-Spektrums einer zu untersuchenden Probe (20) eines alkoholischen Getränks innerhalb eines flaschenartigen geschlossenen Behältnisses (2), mit mindestens einer 2. einen Laser umfassenden optischen Einrichtung zur Erzeugung eines Laserstrahls, mindestens einem motorisierten Filter unterschiedlicher optischer Dichte zur Einstellung der Laserintensität auf der Probe und mindestens einer Linse für Licht im sichtbaren Spektrum, wobei zur Messung der Streuung, Absorption, Transmission und/oder Reflexion ferner ein Mikroskop (11) mit einem Macro-Objektiv (110) mit einer langen Brennweite von mindestens D=10mm und einer Halterung (12) für das daran anordbare, flaschenartige Behältnis (2) vorgesehen ist, welches aus Glas besteht, wobei die Messung innerhalb des unterhalb des Macro-Objektivs (110) des Mikroskops (11) an der Halterung (12) anordbaren, flaschenartigen Behältnisses (2) vorgenommen wird und der einfallende Laserstrahl mit dem Macro-Objektiv (110) auf den interessierenden Probenbereich (20) innerhalb des flaschenartigen Behältnisses (2) fokussiert wird, wobei der Laser eine Wellenlänge von 785 nm umfasst, wobei zur Durchführung des Verfahrens zunächst zwei verschlossene Behältnisse (2) mit Alkoholinhalt nacheinander mit dem Raman-Spektrometer (10) vermessen werden und die gemessenen Spektren miteinander verglichen werden mit Hilfe einer Hauptkomponentenanalyse, und wobei die Echtheit des Alkoholinhalts in den Behältnissen anhand von Übereinstimmungen der Spektren geprüft wird.

2. Verfahren nach Anspruch 1,
**dadurch gekennzeichnet, dass** dass der Brennpunkt in das Glas des flaschenartigen Behältnisses (2) gebracht wird, wobei ein Spektrum des Flaschenglases gewonnen wird, das zur Differenzbildung vom Gesamtspektrum abgezogen werden kann.

3. Vorrichtung (1) zur Durchführung des Verfahrens nach Anspruch 1, umfassend ein Raman-Spekfrometer (10) zur Detektion eines Raman-Spektrums einer zu untersuchenden Probe (20) eines alkoholischen Getränks innerhalb des flaschenartigen geschlossenen Behältnisses (2), mit mindestens einer einen Laser umfassenden optischen Einrichtung zur Erzeugung eines Laserstrahls mit einer Wellenlänge von 785 nm, mindestens einem motorisierten Filter unterschiedlicher optischer Dichte zur Einstellung der Laserintensität auf der Probe und mindestens einer Linse für Licht im sichtbaren Spektrum, wobei die Vorrichtung (1) zur Messung der Streuung, Absorption, Transmission und/oder Reflexion ferner ein Mikroskop (11) mit einer zur Aufnahme des flaschenartigen Behältnisses (2) ausgebildeten Halterung (12) umfasst,
und ferner umfassend
ein Makro-Objektiv mit einer langen Brennweite von mindestens D = 10 mm zur Fokussierung des Laserstrahls auf den interessierenden Probenbereich (20) innerhalb des flaschenartigen Behältnisses (2),
und Mittel zur Durchführung einer Hauptkomponentenanalyse von Raman-Spektren sowie deren Vergleich.

## Claims

1. Procedure for the analysis of alcoholic beverages regarding the validity of their origin by means of a Raman spectrometer (10) for the determination of a Raman spectrum of a sample to be investigated (20) of an alcoholic beverage within a unopened and bottle-shaped vessel (2), using at least one laser optical device to create a laser beam, with at least one motorised filter of varying optical density to adjust the laser beam to the sample and at least one lens for light in the visible spectrum, whereby for the measurements of refraction, absorption, transmission and/or reflection a microscope (11) with a macro objective (1109 with a long focus of at least D=20mm and a fastening device (12) for the fixable bottle-shaped vessel (2) is provided, made of glass, whereby the measurement is made inside bottle-shaped vessel (2) positioned below the macro objective (110) of the microscope (11) in the fixture (12), whereby the laser has a wavelength of 785 nm, and whereby for the execution of the procedure two closed vessels containing an alcoholic beverage (2) are measured subsequently with the Raman spectrometer (10), whereby the measured spectra are compared with one another by using a main component analysis, and whereby the originality of the alcoholic content inside the vessels is assessed using the correspondence and correlation of the respective spectra.

2. Procedure according to claim 1,
**characterized in that**
the focal point is positioned in the glass of the bottle-shaped vessel (2), revealing a spectrum of the glass, which can be deducted from total spectrum to determine a differential.

3. Device (1) for executing the procedure according to claim 1, including a Raman spectrometer (19(for the detection of a Raman spectrum of a sample to be assessed (20) of an alcoholic beverage contained inside a bottle-shaped vessel (2), with at least one laser containing optical devices for the creation of a laser beam with a wavelength of 785 nm, at least one motorised filter of varying optical densities for the adjustment of the laser intensity to the sample and at least one lens for light in the visible spectrum, whereby the device (1) includes a microscope (11) for the measurement of refraction, absorption, transmission and/or reflection with a fixture (12) designed to hold the bottle-shaped vessel,
and furthermore including
a macro objective with a long focal length of at least D=10mm to focus the laser beam on the interesting sample area (20) within the bottle-shaped vessel (2),
and means for performing a main component analysis of the Raman spectrum and their comparison.

## Revendications

1. Procédé d'analyse de l'authenticité de boissons alcoolisées au moyen d'un spectromètre Raman (10) pour détecter un spectre Raman d'un échantillon (20) d'une boisson alcoolisée à examiner à l'intérieur d'un récipient fermé de type bouteille (2), ayant au moins un dispositif optique comprenant un laser pour générer un faisceau laser, au moins un filtre motorisé de densité optique différente pour régler l'intensité du laser sur l'échantillon et au moins une lentille pour la lumière dans le spectre visible, dans lequel, afin de mesurer la diffusion, l'absorption ,transmission et/ou réflexion, un microscope (11) avec un macro-objectif (110) avec une longue distance focale d'au moins D=10mm et un support (12) pour le récipient en forme de bouteille (2) qui peut être disposé sur celui-ci est en outre prévu, qui se compose de verre, la mesure étant effectuée à l'intérieur du récipient en forme de bouteille (2) qui peut être disposé sous le macro-objectif (110) du microscope (11) sur le support (12), et le faisceau laser incident étant dirigé avec le macro-objectif (110) sur la zone d'échantillon (20) intéressante à l'intérieur du récipient en forme de bouteille (2) dans lequel le laser présente une longueur d'onde de 785 nm, dans lequel, pour mettre en oeuvre le procédé, deux récipients scellés (2) avec une teneur en alcool sont d'abord mesurés successivement avec le spectromètre Raman (10) et les spectres mesurés sont comparés les uns aux autres à l'aide d'une analyse en composantes principales, et dans lequel l'authenticité de la teneur en alcool dans les récipients est vérifiée sur la base des correspondances des spectres.

2. Procédé selon la revendication 1,
**caractérisé en ce que**
le point focal est amené dans le verre du récipient en forme de bouteille (2), ce qui permet d'obtenir un spectre du verre de la bouteille qui peut être soustrait du spectre total pour la formation de la différence.

3. Appareil (1) pour la mise en oeuvre du procédé selon la revendication 1, comprenant un spectromètre Raman (10) pour détecter un spectre Raman d'un échantillon (20) d'une boisson alcoolisée à examiner à l'intérieur du récipient fermé en forme de bouteille (2), ayant au moins un dispositif optique comprenant un laser pour générer un faisceau laser avec une longueur d'onde de 785 nm, au moins un filtre motorisé de densité optique différente pour ajuster l'intensité du laser sur l'échantillon, et au moins une lentille pour la lumière dans le spectre visible, dans lequel le dispositif (1) pour mesurer la diffusion, l'absorption, la transmission et/ou la réflexion comprend en outre un microscope (11) ayant un support (12) adapté pour recevoir le récipient en forme de bouteille (2)
et comprenant en outre
un macro-objectif avec une longue distance focale d'au moins D = 10 mm pour focaliser le faisceau laser sur la région d'intérêt de l'échantillon (20) à l'intérieur du récipient en forme de bouteille (2),
et les moyens d'effectuer une analyse en composantes principales des spectres Raman et leur comparaison.
